# EUROPEAN PATENT APPLICATION

(11) **EP 3 881 865 A1**
(43) Date of publication of application: **22.09.2021**
(21) Application number: 19883877.3
(22) Date of filing: 15.11.2019
(51) Int. Cl.: A61K 39/395, A61K 9/08, A61K 47/26, A61K 47/18, A61K 47/22, A61K 9/00, A61K 39/00

(54) **STABLE LIQUID COMPOSITION COMPRISING PROTEIN**

(30) Priority: 16.11.2018 KR 20180141556
(71) Applicant: Samsung Bioepis Co., Ltd., Yeonsu-gu Incheon 21987 (KR)
(72) Inventor: JUNG, Youngseok, Incheon 22008 (KR); HONG, Jahye, Incheon 21982 (KR); JOO, Kyung Hee, Gwangmyeong-si Gyeonggi-do 14258 (KR); LEE, Jaemin, Seoul 05536 (KR); LEE, Hun Joo, Incheon 21996 (KR); KIM, Yongkook, Incheon 22003 (KR)
(74) Representative: Boult Wade Tennant LLP
(86) International application number: PCT/KR2019/015709
(87) International publication number: WO 2020/101452

(57) **Abstract**

Disclosed is a stable liquid formulation of a protein. Provided is a liquid protein composition comprising a protein, and being free of a buffer and/or comprising histidine, and a preparation method thereof.

## Description

### Technical Field

The present disclosure relates to a stable liquid formulation of a protein and to a liquid composition comprising a protein, and free of a buffer and/or comprising histidine, and a preparation method thereof.

### Background Art

An antibody drug, which can be designed for targeting, has the advantage of resulting in fewer side effects than general protein drugs, and greater therapeutic effects even when used in small doses. However, in order to exert such effects, antibody drugs inevitably require huge molecular weights and complex structures. These structural features may lead to physical/chemical instability, which may result in inhibiting the activity of antibody drugs. Thus, it has been required to develop a suitable formulation therefor.

In an antibody drug, the antibody may become unstable due to various factors such as temperature, pH, buffer, concentration excipient, and the like. In order to develop a formulation that is capable of reducing the instability and increasing medicinal quality of the antibody drugs, attempts have been made for buffer modification, pH optimization, stabilizer addition, and so forth.

Denosumab is marketed as two commercial products: one under the trade name of Prolia® for treatment of osteoporosis; and the other under the trade name of Xgeva® for the therapy of cancer. The indications for each product are also different. Prolia® is a liquid formulated antibody drug of 60 mg/mL denosumab, pH 5.2, 17 mM glacial acetic acid buffer, 4.7% sorbitol, and 0.01% polysorbate20. Xgeva® is also a liquid formulated antibody drug formulation of 70 mg/mL denosumab, pH 5.2, 18 mM glacial acetic acid buffer, 4.6% sorbitol, and 0.01% polysorbate20. Although containing the same ingredients, the two products differ from each other with respect to the composition ratio including the antibody content, and the like. In case of the two products of Denosumab, it is required to minimize the instability caused by the difference in the concentration of antibody. Because Prolia® is administered twice per year and the relatively long administration period demands more stable formulation, the more stable formulation of the drug has been developed.

### Detailed Description of the Invention

### Technical Problem

Accordingly, the present disclosure provides a stable liquid pharmaceutical composition of a protein.

An embodiment provides a liquid composition of a protein that comprises a protein, has a pH in a range of 5 to 7, and does not comprise (is free of) at least one selected from the group consisting of acetate, succinate, and glutamate, or does not comprise a buffer. The liquid composition may further comprise histidine. The liquid composition may further comprise a surfactant. The liquid composition may be an aqueous, liquid composition.

In one embodiment, the liquid composition may:
(1) comprise a protein, for example, an antibody such as an anti-RANKL antibody,
(2) have a pH of 5 to 7, and
(3-1) be free of an acetate.

In another embodiment, the liquid composition may:
(1) comprise a protein, for example, an antibody such as an anti-RANKL antibody,
(2) have a pH of 5 to 7, and
(3-2) be free of a succinate.

In another embodiment, the liquid composition may:
(1) comprise a protein, for example, an antibody, such as an anti-RANKL antibody,
(2) have a pH of 5 to 7, and
(3-3) be free of both of an acetate and a succinate.

The liquid composition may be free of glutamate. The liquid composition may further comprise histidine.

In another embodiment, the liquid composition may:
(1) comprise a protein, for example, an antibody such as an anti-RANKL antibody,
(2) have a pH of 5 to 7, and
(3-4) be free of a buffer. In this context, the liquid composition may be free of histidine.

The liquid composition may be an aqueous liquid composition.

The liquid composition may further comprise at least one selected from the group consisting of a sugar, a sugar derivative (a sugar acid, a polyol such as a sugar alcohol, etc.), an amino acid, a pharmaceutically acceptable salt thereof, and the like, in addition to the protein or the protein and the histidine (if contained). In the liquid composition provided in the present disclosure, the at least one selected from the group of a sugar, a sugar derivative, an amino acid, a pharmaceutically acceptable salt thereof, and the like may serve as a stabilizer and/or a tonicity agent.

In one embodiment, when the liquid composition comprises at least one sugar (for example, at least one selected from the group consisting of trehalose, sucrose, mannose, and maltose), the concentration thereof may be in a range of 1 to 15%(w/v), 3 to 15%(w/v), 5 to 15%(w/v), 7 to 15%(w/v), 1 to 10%(w/v), 3 to 10%(w/v), 5 to 10%(w/v), 7 to 10%(w/v), 1 to 7%(w/v), 2 to 7%(w/v), 3 to 7%(w/v), 4 to 7%(w/v), 1 to 5%(w/v), 2 to 5%(w/v), 3 to 5%(w/v), or 4 to 5%(w/v), 4.5 to 5%(w/v) (e.g., about 4.7%(w/v)), or 7.8 to 8.2%(w/v) (e.g., about 7.8%(w/v), about 7.9%(w/v), about 8%(w/v), about 8.1 %(w/v), or about 8.2%(w/v)), based on the total liquid composition;
when the liquid composition comprises at least one sugar alcohol (for example, at least one selected from the group consisting of sorbitol and mannitol), the concentration thereof may be in a range of 1 to 10%(w/v), 2.5 to 10%(w/v), 3 to 10%(w/v), 3.5 to 10%(w/v), 4 to 10%(w/v), 1 to 8%(w/v), 2.5 to 8%(w/v), 3 to 8%(w/v), 3.5 to 8%(w/v), 4 to 8%(w/v), 1 to 6%(w/v), 2.5 to 6%(w/v), 3 to 6%(w/v), 3.5 to 6%(w/v), 4 to 6%(w/v), 4 to 5.5%(w/v), 4 to 5%(w/v), 4.2 to 4.8%(w/v), or 4.4 to 4.7%(w/v) (e.g., about 4.4%(w/v), about 4.5%(w/v), about 4.6%(w/v), or about 4.7%(w/v)), based on the total liquid composition;
when the liquid composition comprises at least one amino acid (for example, at least one selected from the group consisting of arginine (Arg), lysine (Lys), proline (Pro), glycine (Gly), alanine (Ala), methionine (Met), serine (Ser), and pharmaceutically acceptable salts of the amino acids), the concentration thereof may be in a range of 0.1 to 300mM, 0.5 to 300mM, 1 to 300mM, 5 to 300mM, 10 to 300mM, 30 to 300mM, 50 to 300mM, 80 to 300mM, 100 to 300mM, 120 to 300mM, 0.1 to 250mM, 0.5 to 250mM, 1 to 250mM, 5 to 250mM, 10 to 250mM, 30 to 250mM, 50 to 250mM, 80 to 250mM, 100 to 250mM, 120 to 250mM, 0.1 to 200mM, 0.5 to 200mM, 1 to 200mM, 5 to 200mM, 10 to 200mM, 30 to 200mM, 50 to 200mM, 80 to 200mM, 100 to 200mM, 120 to 200mM, 0.1 to 160mM, 0.5 to 160mM, 1 to 160mM, 5 to 160mM, 10 to 160mM, 30 to 160mM, 50 to 160mM, 80 to 160mM, 100 to 160mM, 120 to 160mM, or 130 to 150mM.

In an embodiment, the liquid composition may further comprise a surfactant. When the liquid composition comprises a surfactant, the surfactant may be used at a concentration in a range of 0.001 to 1%(w/v), 0.001 to 0.5%(w/v), 0.001 to 0.1%(w/v), 0.001 to 0.05%(w/v), 0.005 to 1%(w/v), 0.005 to 0.5%(w/v), 0.005 to 0.1%(w/v), 0.005 to 0.05%(w/v), or 0.008 to 0.02%(w/v), based on the total liquid composition. The surfactant may be any one that can be conventionally used in a protein formulation. For example, the surfactant may comprise at least one selected from non-ionic surfactants.

Another embodiment provides a pharmaceutical composition comprising the liquid composition. The pharmaceutical composition has the pharmacological activity corresponding to the activity of the protein contained in the liquid composition.

In the liquid composition or the pharmaceutical composition, for example, the protein may have a molecular weight of 10 to 500 kDa, 10 to 400 kDa, 10 to 300 kDa, 10 to 200 kDa, or 10 to 150 kDa. In an embodiment, the protein may be a RANKL (receptor activator of nuclear factor kappa-B ligand) antagonist (e.g., anti-RANKL antibody), for example, denosumab (molecular weight of about 147 kDa; e.g., PROLIA® or XGEVA®), but not be limited thereto. When the protein is a RANKL antagonist (e.g., anti-RANKL antibody), for example, denosumab, the pharmaceutical composition may be applied to the treatment of osteoporosis and/or cancer/cancer metastasis.

Another embodiment provides a method for stabilizing a protein or for preparing a stable liquid composition of a protein, the method comprising a step of mixing the protein in the composition described above.

### Technical Solution

The present disclosure provides a stable liquid pharmaceutical composition of a protein, and a preparation method thereof.

An embodiment provides a liquid protein composition that comprises a protein, has a pH in a range of 5 to 7, and is free of at least one selected from the group consisting of acetate, succinate, and glutamate, or is free of a buffer. The liquid composition may further comprise histidine. The liquid composition may further comprise a surfactant. The liquid composition may be an aqueous liquid composition.

In one embodiment, the liquid composition may:
(1) comprise a protein, for example, an antibody such as an anti-RANKL antibody,
(2) have a pH of 5 to 7, and
(3-1) be free of an acetate.

In another embodiment, the liquid composition may:
(1) comprise a protein, for example, an antibody such as an anti-RANKL antibody,
(2) have a pH of 5 to 7, and
(3-2) be free of a succinate.

In another embodiment, the liquid composition may:
(1) comprise a protein, for example, an antibody, such as an anti-RANKL antibody,
(2) have a pH of 5 to 7, and
(3-3) be free of both of an acetate and a succinate.

The liquid composition may be free of glutamate.

The liquid composition may further comprise histidine.

In another embodiment, the liquid composition may:
(1) comprise a protein, for example, an antibody such as an anti-RANKL antibody,
(2) have a pH of 5 to 7, and
(3-4) be free of a buffer. In this context, the liquid composition may be free of histidine.

The liquid composition may be an aqueous liquid composition.

The liquid composition may have a pH in a range of 4 to 8, for example, 4.5 to 7, 4.8 to 7, 5 to 7, 5.2 to 7, 4.5 to 6.8, 4.8 to 6.8, 5 to 6.8, 5.2 to 6.8, 4.5 to 6.5, 4.8 to 6.5, 5 to 6.5, 5.2 to 6.5, 4.5 to 6.3, 4.8 to 6.3, 5 to 6.3, 5.2 to 6.3, 4.5 to 6, 4.8 to 6, 5 to 6, 5.2 to 6,4.5 to 5.8, 4.8 to 5.8, 5 to 5.8, 5.2 to 5.8, 4.5 to 5.6,4.8 to 5.6, 5 to 5.6, 5.2 to 5.6,4.9 to 5.5,4.9, 5.0, 5.1, 5.2, 5.3, 5.4, or 5.5.

The liquid composition of a protein provided in the present disclosure may be free of either or both of an acetate and a succinate, may also be free of a glutamate, and/or may comprise histidine, whereby the pH, which is one of the important factors affecting protein stability, can be maintained within the suitable range stated above and the protein in the composition can be of excellent stability (for example, excellent protein stability indicators such as high molecular weight (%HMW), % charge variant (e.g., an amount of acidic variant (%Acidic)).

As used herein, the description such as "the liquid composition of a protein provided by the present disclosure is free of ingredient A" is intended to mean that the composition does not comprise ingredient A (ingredient A is absent in the composition) or does not substantially comprise ingredient A (ingredient A is substantially absent in the composition). In this regard, the description "ingredient A is not substantially comprised or is absent in the composition" may be understood as "ingredient A is not comprised (or is absent) at all" or "ingredient A is comprised (or is present) in an amount that is too small to allow the exhibition of the intended pharmaceutical function or to be detected in the liquid composition of a protein. In an embodiment, the term "free of ingredient A" may mean that ingredient A is not detected by a conventional method.

Hereinafter, a detailed description will be given of the liquid composition provided by the present disclosure:

### 1. Protein

In the liquid composition provided by the present disclosure, the protein may be a protein drug, for example, a protein, e.g., an antibody, with a molecular weight of 10 to 500 kDa, 10 to 400 kDa, 10 to 300 kDa, 10 to 200 kDa, or 10 to 150 kDa.

Any antibody may be used as long as it has two heavy chains connected to each other by disulfide bonds wherein each heavy chain is connected to respective light chain by a disulfide bond. The antibody may be a naturally occurring one or a recombinantly/chemically synthesized (non-naturally obtained) one. As used herein, the term "antibody" is intended to encompass, without limitation, any conventional antibody and/or an antigen-binding fragment or single chain thereof, any protein or peptide molecule comprising at least a portion of an immunoglobulin molecule having a biological activity of binding to an antigen. Hence, the antibody may comprise a single domain antibody or a fragment thereof, for example, a complementarity-determining region (CDR) in a heavy chain or light chain of a single domain antibody or a ligand-binding portion thereof, a heavy chain or light chain variable region, a heavy chain or light chain constant region, a framework region (FR), and/or any portion thereof, and/or at least a portion of a binding protein. In an embodiment, the antibody may be a monoclonal antibody. In an embodiment, the antibody may be an animal-derived antibody, a chimeric antibody, a humanized antibody, or a human antibody. In an embodiment, the antibody may include a mono-, bi- or multispecific antibody.

In an embodiment, the protein may be a RANKL (Receptor activator of nuclear factor kappa-B ligand) antagonist (e.g., anti-RANKL antibody), for example, denosumab (molecular weight: about 147 kDa; e.g., PROLIA®, XGEVA®), but not be limited thereto. When the protein is a RANKL antagonist (e.g., anti-RANKL antibody), for example, denosumab, the pharmaceutical composition may be applied to the treatment of osteoporosis and/or cancer.

The protein contained in the liquid composition may be naturally occurring or non-naturally occurring, for example, may be produced in a recombinant or synthetic manner.

The liquid composition may comprise the protein at a concentration of 5 mg/ml to 300 mg/ml, 5 mg/ml to 250 mg/ml, 5 mg/ml to 200 mg/ml, 5 mg/ml to 150 mg/ml, 5 mg/ml to 125 mg/ml, 5 mg/ml to 100 mg/ml, 5 mg/ml to 90 mg/ml, 5 mg/ml to 80 mg/ml, 5 mg/ml to 70 mg/ml, 5 mg/ml to 60 mg/ml, 10 mg/ml to 300 mg/ml, 10 mg/ml to 250 mg/ml, 10 mg/ml to 200 mg/ml, 10 mg/ml to 150 mg/ml, 10 mg/ml to 125 mg/ml, 10 mg/ml to 100 mg/ml, 10 mg/ml to 90 mg/ml, 10 mg/ml to 80 mg/ml, 10 mg/ml to 70 mg/ml, 10 mg/ml to 60 mg/ml, 20 mg/ml to 300 mg/ml, 20 mg/ml to 250 mg/ml, 20 mg/ml to 200 mg/ml, 20 mg/ml to 150 mg/ml, 20 mg/ml to 125 mg/ml, 20 mg/ml to 100 mg/ml, 20 mg/ml to 90 mg/ml, 20 mg/ml to 80 mg/ml, 20 mg/ml to 70 mg/ml, 20 mg/ml to 60 mg/ml, 30 mg/ml to 300 mg/ml, 30 mg/ml to 250 mg/ml, 30 mg/ml to 200 mg/ml, 30 mg/ml to 150 mg/ml, 30 mg/ml to 125 mg/ml, 30 mg/ml to 100 mg/ml, 30 mg/ml to 90 mg/ml, 30 mg/ml to 80 mg/ml, 30 mg/ml to 70 mg/ml, 30 mg/ml to 60 mg/ml, 40 mg/ml to 300 mg/ml, 40 mg/ml to 250 mg/ml, 40 mg/ml to 200 mg/ml, 40 mg/ml to 150 mg/ml, 40 mg/ml to 125 mg/ml, 40 mg/ml to 100 mg/ml, 40 mg/ml to 90 mg/ml, 40 mg/ml to 80 mg/ml, 40 mg/ml to 70 mg/ml, 40 mg/ml to 60 mg/ml, 50 mg/ml to 300 mg/ml, 50 mg/ml to 250 mg/ml, 50 mg/ml to 200 mg/ml, 50 mg/ml to 150 mg/ml, 50 mg/ml to 125 mg/ml, 50 mg/ml to 100 mg/ml, 50 mg/ml to 90 mg/ml, 50 mg/ml to 80 mg/ml, 50 mg/ml to 70 mg/ml, 50 mg/ml to 60 mg/ml, 60 mg/ml to 300 mg/ml, 60 mg/ml to 250 mg/ml, 60 mg/ml to 200 mg/ml, 60 mg/ml to 150 mg/ml, 60 mg/ml to 125 mg/ml, 60 mg/ml to 100 mg/ml, 60 mg/ml to 90 mg/ml, 60 mg/ml to 80 mg/ml, or 60 mg/ml to 70 mg/ml, for example, at a concentration of 50 mg/ml, 55 mg/ml, 60 mg/ml, 65 mg/ml, 70 mg/ml, 75 mg/ml, or 80 mg/ml.

### 2. Buffer

The liquid composition provided in the present disclosure may be free of a buffer. In an embodiment, the buffer that is not comprised in (is excluded from) the liquid composition may be at least one of conventionally used buffers having a buffering capacity. For example, the buffer that is not comprised in the liquid composition may comprise at least one selected from the group consisting of (1) at least one acid selected from phosphoric acid, acetic acid, citric acid, succinic acid, and carbonic acid, and ionic forms thereof (e.g., phosphate, acetate, citrate, succinate, carbonate, etc.); (2) pharmaceutically acceptable salts thereof (e.g., sodium salt, potassium salt, etc.), (3) at least one amino acid selected from glutamic acid and aspartic acid, and ionic forms thereof (e.g., glutamate, aspartate, etc.), and (4) pharmaceutically acceptable salts of the amino acids (e.g., hydrochloride salt, etc.).

Unless stated otherwise in the present disclosure, the acids of (1) and/or the amino acids of (3), and ionic forms thereof have equivalent meanings to each other, or may be used interchangeably.

The liquid composition may not comprise at least one selected from the group consisting of: phosphoric acid, acetic acid (e.g., glacial acetic acid), citric acid, succinic acid, carbonic acid, ionic forms (e.g., phosphate, acetate, citrate, succinate, carbonate, etc.) of the acids (phosphoric acid, acetic acid, citric acid, succinic acid, carbonic acid, etc.), pharmaceutically acceptable salts (e.g., sodium salt, potassium salt, etc.) of the acids (phosphoric acid, acetic acid, citric acid, succinic acid, and carbonic acid); and aspartic acid, glutamic acid, ionic forms (e.g., glutamate, aspartate, etc.) of the amino acids (aspartic acid, glutamic acid), and pharmaceutically acceptable salts (e.g., hydrochloride salt, etc.) of the amino acids (aspartic acid, glutamic acid). The amino acid that is not comprised in the liquid composition may be not an amino acid present in the protein, but a free amino acid.

In an embodiment, the liquid composition may be free of acetic acid and/or acetate.

In another embodiment, the liquid composition may be free of succinic acid and/or succinate.

In another embodiment, the liquid composition may be free of acetic acid, acetate, succinic acid, and succinate.

In another embodiment, the liquid composition may be free of glutamic acid and/or glutamate.

In another embodiment, the liquid composition may be free of acetic acid, acetate, glutamic acid, and glutamate.

In another embodiment, the liquid composition may be free of succinic acid, succinate, glutamic acid, and glutamate.

In another embodiment, the liquid composition may be free of acetic acid, acetate, succinic acid, succinate, glutamic acid, and glutamate.

In another embodiment, the liquid composition may be free of acetic acid, acetate, succinic acid, succinate, glutamic acid, glutamate, phosphoric acid, phosphate, citric acid, citrate, carbonic acid, carbonate, aspartic acid, and aspartate.

### 3. Histidine

The liquid composition provided in the present disclosure may comprise histidine. In the liquid composition, histidine may function as a buffer. In the liquid composition, histidine may also function as a stabilizer for the protein. For example, histidine may suppress an acidic variants of the protein (inhibition of formation of an acidic variant) or serve as an acidic variants inhibitor of the protein. In the liquid composition, histidine may suppress the acid variants of the protein (inhibit the formation of an acidic variant, reduce %Acidic increment). Moreover, histidine may have the effect of inhibiting protein aggregation (e.g., inhibit formation of high molecular weight (HMW), reduce %HMW increment). Hence, the liquid composition may comprise histidine as a buffer, and/or an acidic variants inhibitor and/or a protein aggregation inhibitor.

In the liquid composition, histidine may be in isolated free amino acid form (that is, is not a histidine residue of the protein).

If the liquid composition comprises histidine, the concentration of histidine in the liquid composition may be in a range of 1 to 100mM, 1 to 70mM, 1 to 50mM, 1 to 40mM, 1 to 30mM, 1 to 25mM, 1 to 22mM, 1 to 20mM, 5 to 100mM, 5 to 70mM, 5 to 50mM, 5 to 40mM, 5 to 30mM, 5 to 25mM, 5 to 22mM, 5 to 20mM, 10 to 100mM, 10 to 70mM, 10 to 50mM, 10 to 40mM, 10 to 30mM, 10 to 25mM, 10 to 22mM, 10 to 20mM, 14 to 100mM, 14 to 70mM, 14 to 50mM, 14 to 40mM, 14 to 30mM, 14 to 25mM, 14 to 22mM, 14 to 20mM, 16 to 100mM, 16 to 70mM, 16 to 50mM, 16 to 40mM, 16 to 30mM, 16 to 25mM, 16 to 22mM, or 16 to 20mM; for example, 16mM, 17mM, 18mM, 19mM, or 20mM.

### 4. Sugar, Sugar Derivative, and/or Amino Acid

The liquid composition may comprise at least one selected from the group consisting of sugars, sugar derivatives, and amino acids.

The at least one selected from the group consisting of sugars, sugar derivatives, and amino acids may serve as a stabilizer and/or a tonicity agent for the protein in the liquid composition, but not be limited thereto. In this regard, the amino acid that can be comprised as a stabilizer and/or a tonicity agent may be at least one amino acid, except for histidine (that is, the amino acid may not include histidine).

### (1) Sugar and Sugar Derivative (sugar acid, polyol such as sugar alcohol)

In an embodiment, the sugar may be at least one selected from the group consisting of a monosaccharide, a disaccharide, an oligosaccharide, and a polysaccharide, but is not limited thereto. The monosaccharide may be at least one selected from the group consisting of glucose, fructose, galactose, mannose, and the like, but is not limited thereto. The disaccharide may be at least one selected from the group consisting of sucrose, lactose, maltose, trehalose, and the like, but is not limited thereto. The oligosaccharide may be at least one selected from the group consisting of fructooligosaccharide, galactooligosaccharide, mannanoligosaccharide, and the like, but is not limited thereto. The polysaccharide may be at least one selected from the group consisting of starch, glycogen, cellulose, chitin, pectin, and the like, but is not limited thereto.

The sugar acid may be at least one selected from the group consisting of aldonic acid (glyceric acid, etc.), ulosonic acid (neuraminic acid, etc.), uronic acid (glucuronic acid, etc.), aldaric acid (tartaric acid, etc.), and the like, but is not limited thereto.

The sugar alcohol may be at least one selected from the group consisting of glycerol, erythritol, threitol, arabitol, xylitol, ribitol, mannitol, sorbitol, galactitol, fucitol, iditol, inositol, volemitol, isomalt, maltitol, lactitol, maltotritol, maltotetraitol, polyglycitol, and the like, but is not limited thereto.

In an embodiment, the liquid composition may comprise at least one sugar or sugar alcohol selected from the group consisting of trehalose, sucrose, mannose, sorbitol, maltose, mannitol, and the like (for example, at least one selected from the group consisting of sorbitol, sucrose, trehalose, and the like).

In an embodiment, when the liquid composition comprises at least one sugar (for example, at least one selected from the group consisting of trehalose, sucrose, mannose, and maltose), the concentration thereof may be in a range of 1 to 15%(w/v), 3 to 15%(w/v), 5 to 15%(w/v), 7 to 15%(w/v), 1 to 10%(w/v), 3 to 10%(w/v), 5 to 10%(w/v), 7 to 10%(w/v), 1 to 7%(w/v), 2 to 7%(w/v), 3 to 7%(w/v), 4 to 7%(w/v), 1 to 5%(w/v), 2 to 5%(w/v), 3 to 5%(w/v), 4 to 5%(w/v), 4.5 to 5%(w/v) (e.g., about 4.7%(w/v)) or 7.8 to 8.2%(w/v) (e.g., about 8%(w/v)) (for example, trehalose or sucrose may be contained in an amount of 7.8 to 8.2%(w/v) (e.g., about 7.8%(w/v), about 7.9%(w/v), about 8%(w/v), about 8.1%(w/v), or about 8.2%(w/v))), based on the total liquid composition; and
when the liquid composition comprises at least one sugar alcohol (for example, at least one selected from the group consisting of sorbitol, and mannitol), the concentration thereof may be in a range of 1 to 10%(w/v), 2.5 to 10%(w/v), 3 to 10%(w/v), 3.5 to 10%(w/v), 4 to 10%(w/v), 1 to 8%(w/v), 2.5 to 8%(w/v), 3 to 8%(w/v), 3.5 to 8%(w/v), 4 to 8%(w/v), 1 to 6%(w/v), 2.5 to 6%(w/v), 3 to 6%(w/v), 3.5 to 6%(w/v), 4 to 6%(w/v), 4 to 5.5%(w/v), 4 to 5%(w/v), 4.2 to 4.8%(w/v), or 4.4 to 4.7%(w/v) (e.g., about 4.4%(w/v), about 4.5%(w/v), about 4.6%(w/v)), or about 4.7%(w/v)), based on the total liquid composition.

In an embodiment, the liquid composition may comprise:
at least one sugar selected from the group consisting of trehalose, sucrose, mannose, and maltose at a concentration of 1 to 15%(w/v), 3 to 15%(w/v), 5 to 15%(w/v), 7 to 15%(w/v), 1 to 10%(w/v), 3 to 10%(w/v), 5 to 10%(w/v), 7 to 10%(w/v), 1 to 7%(w/v), 2 to 7%(w/v), 3 to 7%(w/v), 4 to 7%(w/v), 1 to 5%(w/v), 2 to 5%(w/v), 3 to 5%(w/v), 4 to 5%(w/v), 4.5 to 5%(w/v) (e.g., about 4.7%(w/v)) or 7.8 to 8.2%(w/v) (e.g., about 7.8%(w/v), about 7.9%(w/v), about 8%(w/v), about 8.1 %(w/v), or about 8.2%(w/v)), based on the total liquid composition thereof;
at least one sugar alcohol selected from the group consisting of sorbitol and mannitol at a concentration of 1 to 10%(w/v), 2.5 to 10%(w/v), 3 to 10%(w/v), 3.5 to 10%(w/v), 4 to 10%(w/v), 1 to 8%(w/v), 2.5 to 8%(w/v), 3 to 8%(w/v), 3.5 to 8%(w/v), 4 to 8%(w/v), 1 to 6%(w/v), 2.5 to 6%(w/v), 3 to 6%(w/v), 3.5 to 6%(w/v), 4 to 6%(w/v), 4 to 5.5%(w/v), 4 to 5%(w/v), 4.2 to 4.8%(w/v), or 4.4 to 4.7%(w/v) (e.g., about 4.4%(w/v), about 4.5%(w/v), about 4.6%(w/v), or about 4.7%(w/v)), based on the total liquid composition thereof; or
a combination thereof.

More particularly, the liquid composition may comprise:
trehalose or sucrose at a concentration of 1 to 15%(w/v), 3 to 15%(w/v), 4 to 15%(w/v), 5 to 15%(w/v), 7 to 15%(w/v), 1 to 10%(w/v), 3 to 10%(w/v), 4 to 10%(w/v), 5 to 10%(w/v), 7 to 10%(w/v), 1 to 7%(w/v), 3 to 7%(w/v), 4 to 7%(w/v), 1 to 5%(w/v), 3 to 5%(w/v), 4 to 5%(w/v), 4.2 to 5%(w/v), 4.5 to 5%(w/v), 7 to 9%(w/v), 7 to 8.5%(w/v), 7 to 8.2%(w/v), 7.5 to 9%(w/v), 7.5 to 8.5%(w/v), 7.5 to 8.2%(w/v), 7.8 to 9%(w/v), 7.8 to 8.5%(w/v), 7.8 to 8.2%(w/v), 4.7(w/v), 7.8%(w/v), 7.9%(w/v), 8%(w/v), 8.1 %(w/v), or 8.2%(w/v), based on the total liquid composition thereof;
sorbitol at a concentration of 1 to 10%(w/v), 2.5 to 10%(w/v), 3 to 10%(w/v), 3.5 to 10%(w/v), 4 to 10%(w/v), 1 to 8%(w/v), 2.5 to 8%(w/v), 3 to 8%(w/v), 3.5 to 8%(w/v), 4 to 8%(w/v), 1 to 6%(w/v), 2.5 to 6%(w/v), 3 to 6%(w/v), 3.5 to 6%(w/v), 4 to 6%(w/v), 4 to 5.5%(w/v), 4 to 5.2%(w/v), 4 to 5%(w/v), 4 to 4.8%(w/v), 4.1 to 5.5%(w/v), 4.1 to 5.2%(w/v), 4.1 to 5%(w/v), 4.1 to 4.8%(w/v), 4.1 to 4.7%(w/v), 4.2 to 5.5%(w/v), 4.2 to 5.2%(w/v), 4.2 to 5%(w/v), 4.2 to 4.8%(w/v), 4.2 to 4.7%(w/v), 4.4%(w/v), 4.5%(w/v), 4.6%(w/v), or 4.7%(w/v), based on the total liquid composition thereof; or
a combination thereof.

### (2) Amino acid

As used in the present disclosure, the term "amino acid" may refer to at least one selected from the amino acid per se, an ionic form thereof, and a pharmaceutically acceptable salt thereof. The amino acid may be in an L-form, D-form or in a racemic mixture, and for example, may be in an L-form, but is not limited thereto.

The amino acid that is comprised as a stabilizer and/or a tonicity agent may be at least one selected from amino acids except histidine, and pharmaceutically acceptable salts thereof. For example, the amino acid may be at least one selected from the group consisting of alanine (Ala), arginine (Arg), asparagine (Asn), aspartic acid (Asp), glutamine (Gin), glutamic acid (Glu), glycine (Gly), isoleucine (lie), leucine (Leu), lysine (Lys), methionine (Met), phenylalanine (Phe), proline (Pro), serine (Ser), threonine (Thr), tryptophan (Trp), tyrosine (Tyr), valine (Val), cysteine (Cys), and pharmaceutically acceptable salts thereof (e.g., hydrochloride salt, acetyl amino acid salt, etc.). The amino acid may not be an amino acid present within the protein provided in the present disclosure, but a free amino acid.

In an embodiment, the amino acid may include at least one selected from the group consisting of arginine (Arg), lysine (Lys), proline (Pro), glycine (Gly), alanine (Ala), methionine (Met), serine (Ser), and pharmaceutically acceptable salts of the amino acids (for example, hydrochloride salt, acetyl amino acid salt, etc.).

In an embodiment, the liquid composition may comprise at least one amino acid (except histidine; for example, at least one selected from the group consisting of arginine (Arg), lysine (Lys), proline (Pro), glycine (Gly), alanine (Ala), methionine (Met), serine (Ser), and pharmaceutically acceptable salts of the amino acids). In this regard, the concentration of the amino acid in the liquid composition may be 0.1 to 300mM, 0.5 to 300mM, 1 to 300mM, 5 to 300mM, 10 to 300mM, 30 to 300mM, 50 to 300mM, 80 to 300mM, 100 to 300mM, 120 to 300mM, 0.1 to 250mM, 0.5 to 250mM, 1 to 250mM, 5 to 250mM, 10 to 250mM, 30 to 250mM, 50 to 250mM, 80 to 250mM, 100 to 250mM, 120 to 250mM, 0.1 to 200mM, 0.5 to 200mM, 1 to 200mM, 5 to 200mM, 10 to 200mM, 30 to 200mM, 50 to 200mM, 80 to 200mM, 100 to 200mM, 120 to 200mM, 0.1 to 160mM, 0.5 to 160mM, 1 to 160mM, 5 to 160mM, 10 to 160mM, 30 to 160mM, 50 to 160mM, 80 to 160mM, 100 to 160mM, 120 to 160mM, or 130 to 150mM.

In an embodiment, the liquid composition may comprise at least one amino acid selected from the group consisting of arginine (Arg), lysine (Lys), proline (Pro), glycine (Gly), alanine (Ala), methionine (Met), serine (Ser), and pharmaceutically acceptable salts of the amino acids at a concentration of 80 to 200mM, 100 to 200mM, 130 to 200mM, 80 to 150mM, 100 to 150mM, or 120 to 160mM.

More specifically, the liquid composition may comprise arginine (Arg) or arginine hydrochloride (Arg-HCI) at a concentration of 80 to 200mM, 100 to 200mM, 120 to 200mM, 80 to 160mM, 100 to 160mM, 120 to 160mM, 120 to 155mM, 120 to 150mM, 120 to 145mM, 130 to 160mM, 130 to 155mM, 130 to 150mM, 130 to 145mM, 130 to 142mM, 135 to 160mM, 135 to 155mM, 135 to 150mM, 135 to 145mM, 135 to 142mM, 138 to 160mM, 138 to 155mM, 138 to 150mM, 138 to 145mM, 138 to 142mM, or 140mM.

### 5. Sodium chloride and other ingredients

In an embodiment, the liquid composition may be free of sodium chloride. The sodium chloride may function as a stabilizer and/or a tonicity agent. In another embodiment, the liquid composition may further comprise sodium chloride in addition to the sugar, the sugar derivative, and/or the amino acid described above. In another embodiment, the liquid composition does not comprise sodium chloride alone, as a stabilizer and/or a tonicity agent. That is, when the liquid composition comprises sodium chloride, at least one selected from a sugar, a sugar derivative (sugar acid, polyol such as sugar alcohol), an amino acid, and a pharmaceutically acceptable salt thereof, as described above, may also be comprised in the liquid composition.

In another embodiment, the liquid composition may not comprise polyethylene glycol (e.g., PEG3350), a cyclodextrin derivative (e.g., sulfobutylether-β-cyclodextrin), and the like, as a stabilizer.

### 6. Surfactant

In an embodiment, the liquid composition may further comprise a surfactant. Given in the liquid composition, the surfactant may be comprised at the concentration of 0.001 to 1%(w/v), 0.001 to 0.5%(w/v), 0.001 to 0.1%(w/v), 0.001 to 0.05%(w/v), 0.005 to 1%(w/v), 0.005 to 0.5%(w/v), 0.005 to 0.1%(w/v), 0.005 to 0.05%(w/v), or 0.008 to 0.02%(w/v), based on the total liquid composition.

The surfactant may be any surfactant which is pharmaceutically acceptable and capable of evenly dispersing a protein in a liquid composition medium. The surfactant may be a non-ionic surfactant, for example, at least one selected from the group consisting of polysorbates (e.g., polysorbate 20 (polyoxyethylene(20) sorbitan monolaurate), polysorbate 40 (polyoxyethylene(20) sorbitan monopalmitate), polysorbate 60 (polyoxyethylene(20) sorbitan monostearate), polysorbate 80 (polyoxyethylene(20) sorbitan monooleate); the numeral (20) following polyoxyethylene indicates a total number of oxyethylene units (-(CH₂CH₂O)-)), a poloxamer (PEO-PPO-PEO copolymer; PEO: poly(ethylene oxide), PPO: polypropylene oxide)), a polyethylene-polypropylene glycol, a polyoxyethylene compound (e.g., polyoxyethylene-stearate, polyoxyethylene alkyl ether (alkyl: C1-C30), a polyoxyethylene monolauryl ether, an alkylphenyl polyoxyethylene copolymer (alkyl: C1-C30) etc.), sodium dodecyl sulphate (SDS), and the like. For example, the surfactant may be a polysorbate (e.g., polysorbate 20).

### 7. Liquid composition

The liquid composition may comprise a protein; a sugar, a sugar derivative, and/or an amino acid; histidine (if comprised), and a surfactant (if comprised) in the respective aforementioned concentration, and the balance of an aqueous medium (e.g., water (purified water), a saline solution, an injection solution, etc.)

The liquid composition provided in the present disclosure may be isotonic with a living body. For example, the liquid composition may have an osmotic pressure in a range of about 200 mOsm/kg to about 450 mOsm/kg, about 200 mOsm/kg to about 400 mOsm/kg, about 200 mOsm/kg to about 350 mOsm/kg, about 200 mOsm/kg to about 330 mOsm/kg, about 250 mOsm/kg to about 450 mOsm/kg, about 250 mOsm/kg to about 400 mOsm/kg, about 250 mOsm/kg to about 350 mOsm/kg, about 250 mOsm/kg to about 330 mOsm/kg, about 270 mOsm/kg to about 450 mOsm/kg, about 270 mOsm/kg to about 400 mOsm/kg, 270 mOsm/kg to about 350 mOsm/kg, 270 mOsm/kg to about 330 mOsm/kg, about 300 mOsm/kg to about 450 mOsm/kg, about 300 mOsm/kg to about 400 mOsm/kg, about 300 mOsm/kg to about 350 mOsm/kg, or about 300 mOsm/kg to about 330 mOsm/kg, but without limitations thereto. The osmotic pressure may be adjusted by the stabilizer.

The liquid composition provided in the present disclosure may have an electrical conductivity in a range of about 0 mS/cm or greater (or more than 0 mS/cm), about 0.0001 mS/cm or greater, or about 0.001 mS/cm or greater, for example, about 0 mS/cm to about 10 mS/cm, about 0 mS/cm to about 7 mS/cm, 0 mS/cm to about 5 mS/cm, about 0 mS/cm to about 2.5 mS/cm, about 0 mS/cm to about 1 mS/cm, about 0 mS/cm to about 0.5 mS/cm, about 0 mS/cm to about 0.1 mS/cm, about 0 mS/cm to about 0.05 mS/cm, more than about 0 mS/cm and about 10 mS/cm or less, more than about 0 mS/cm and about 7 mS/cm or less, more than 0 mS/cm and about 5 mS/cm or less, more than about 0 mS/cm and about 2.5 mS/cm or less, more than about 0 mS/cm and about 1 mS/cm or less, more than about 0 mS/cm and about 0.5 mS/cm or less, more than about 0 mS/cm and about 0.1 mS/cm or less, about more than 0 mS/cm and about 0.05 mS/cm or less, about 0.0001 mS/cm to about 10 mS/cm, about 0.0001 mS/cm to about 7 mS/cm, 0.0001 mS/cm to about 5 mS/cm, about 0.0001 mS/cm to about 2.5 mS/cm, about 0.0001 mS/cm to about 1 mS/cm, about 0.0001 mS/cm to about 0.5 mS/cm, about 0.0001 mS/cm to about 0.1 mS/cm, about 0.0001 mS/cm to about 0.05 mS/cm, about 0.001 mS/cm to about 10 mS/cm, about 0.001 mS/cm to about 7 mS/cm, 0.001 mS/cm to about 5 mS/cm, about 0.001 mS/cm to about 2.5 mS/cm, 0.001 mS/cm to about 1 mS/cm, about 0.001 mS/cm to about 0.5 mS/cm, about 0.001 mS/cm to about 0.1 mS/cm, or about 0.001 mS/cm to about 0.05 mS/cm.

The liquid composition provided by the present disclosure can be maintained stably for 4 weeks or longer at high temperature of about 40°C.

As used herein, the expression "excellent stability" or "maintained stably" in association with the liquid composition means that the protein in the composition retains the structure, and/or physical, chemical, and/or biological properties thereof during storage (e.g., low high molecular weight, low protein aggregation rate, low protein degradation rate, and/or low acidic variant amount, during storage). Various analyses for evaluating protein stability are well known in the related field. The liquid composition provided in the present disclosure, which is free of a buffer or comprises histidine, may exhibit a low high molecular weight (protein aggregation rate) and/or a low acidic variant amount (acidic variants rate), compared with the case comprising a buffer other than histidine, for example, comprising an acetate or a succinate.

For example, when the liquid composition provided in the present disclosure comprises a protein (antibody) at a concentration of 60 mg/ml, 70 mg/ml, or 80 mg/ml, variation in protein aggregate amount or aggregation rate (High Molecular Weight %(w/v); %HMW) during storage at 40°C for one week or longer, for example, one, two, three, or four weeks (e.g., the variation may refer to Δ%HMW; %HMW at week 4 - %HMW at week 0 (initial)) may be about 5% or less, for example, about 4.5% or less, about 4% or less, about 3.5% or less, about 3% or less, about 2.5% or less, about 2% or less, about 1.5% or less, about 1% or less, about 0.9% or less, about 0.8% or less, about 0.7% or less, about 0.65% or less, about 0.6% or less, about 0.55% or less, about 0.5% or less, about 0.45% or less, or about 0.4% or less, as measured by conventional SEC (size exclusion chromatography), but not be limited thereto.

In another embodiment, when the liquid composition provided in the present disclosure comprises a protein (antibody) at a concentration of 60 mg/ml, 70 mg/ml, or 80 mg/ml, variation in protein aggregate amount or aggregation rate (High Molecular Weight %(w/v); %HMW) during storage at 40°C for one week or longer, for example, one, two, three, or four weeks (e.g., the variation may refer to Δ%HMW; %HMW at week 4 - %HMW at week 0 (initial)) may be lower than that of a liquid composition which is identical to the liquid composition of the present disclosure except for comprising at least one selected from an acetate, a succinate, and a glutamate as a buffer and/or not comprising histidine, by about 0.01% or greater, about 0.5% or greater, about 0.1% or greater, about 0.15% or greater, about 0.2% or greater, about 0.25% or greater, about 0.3% or greater, about 0.35% or greater, about 0.4% or greater, about 0.45% or greater, about 0.5% or greater, about 0.55% or greater, about 0.6% or greater, about 0.65% or greater, about 0.7% or greater, about 0.75% or greater, about 0.8% or greater, about 0.85% or greater, about 0.9% or greater, about 0.95% or greater, or about 1% or greater, for example, by about 0.01% to about 2%, about 0.05% to about 2%, about 0.1% to about 2%, about 0.15% to about 2%, about 0.2% to about 2%, about 0.25% to about 2%, about 0.3% to about 2%, about 0.35% to about 2%, about 0.4% to about 2%, about 0.45% to about 2%, about 0.5% to about 2%, about 0.55% to about 2%, about 0.6% to about 2%, about 0.65% to about 2%, about 0.7% to about 2%, 0.75% to about 2%, 0.8% to about 2%, 0.85% to about 2%, 0.9% to about 2%, 0.95% to about 2%, or 1% to about 2%, as measured by conventional SEC (size exclusion chromatography), but not be limited thereto.

In another embodiment, when the liquid composition provided in the present disclosure comprises a protein (antibody) at a content of 60 mg/ml, 70 mg/ml, or 80 mg/ml, variation in acidic variant amount (%Acidic) during storage at 40°C for one week or longer, for example, one, two, three, or four weeks e.g., the variation may refer to Δ%Acidic; %Acidic at week 4 - %Acidic at week 0 (initial)) may be about 30.0% or less, about 28.0% or less, about 25.0% or less, about 23% or less, about 20% or less, about 18% or less, about 15% or less, about 13% or less, about 10% or less, about 9% or less, about 8% or less, about 7.5% or less, about 7.2% or less, about 7% or less, about 6.9% or less, about 6.8% or less, about 6.7% or less, about 6.6% or less, about 6.5% or less, about 6.4% or less, about 6.3% or less, about 6.2% or less, about 6.1% or less, about 6.0% or less, about 5.9% or less, about 5.8% or less, about 5.7% or less, about 5.6% or less, about 5.5% or less, about 5.4% or less, about 5.3% or less, about 5.2% or less, about 5.1% or less, or about 5.0% or less, as measured by conventional CEX (cation exchange chromatography), but not limited thereto.

In another embodiment, when the liquid composition provided in the present disclosure comprises a protein (antibody) at a content of 60 mg/ml, 70 mg/ml, or 80 mg/ml, variation in acidic variant amount (%Acidic) during storage at 40°C for one week or longer, for example, one, two, three, or four weeks (e.g., the variation may refer to Δ%Acidic; %Acidic at week 4 - %Acidic at week 0 (initial)), which is lower than that of a liquid composition which is identical to the liquid composition of the present disclosure, except for comprising at least one selected from an acetate, a succinate, and a glutamate as a buffer and/or not comprising histidine, by about 0.01 % or greater, about 0.5% or greater, about 0.1% or greater, about 0.15% or greater, about 0.2% or greater, about 0.25% or greater, about 0.3% or greater, about 0.35% or greater, about 0.4% or greater, about 0.45% or greater, about 0.5% or greater, about 0.55% or greater, about 0.6% or greater, about 0.65% or greater, about 0.7% or greater, about 0.75% or greater, about 0.8% or greater, about 0.85% or greater, about 0.9% or greater, about 0.95% or greater, or about 1% or greater, for example, about 0.01% to about 2%, about 0.05% to about 2%, about 0.1% to about 2%, about 0.15% to about 2%, about 0.2% to about 2%, about 0.25% to about 2%, about 0.3% to about 2%, about 0.35% to about 2%, about 0.4% to about 2%, about 0.45% to about 2%, about 0.5% to about 2%, about 0.55% to about 2%, about 0.6% to about 2%, about 0.65% to about 2%, about 0.7% to about 2%, 0.75% to about 2%, 0.8% to about 2%, 0.85% to about 2%, 0.9% to about 2%, 0.95% to about 2%, or 1 % to about 2%, as measured by conventional CEX (cation exchange chromatography), without limitations thereto.

### 8. Pharmaceutical composition

Another embodiment provides a pharmaceutical composition comprising the liquid composition. When the liquid composition comprises, for example, an anti-RANKL antibody as the protein, the pharmaceutical composition may have a prophylactic or therapeutic effect on osteoporosis, cancer or cancer metastasis (e.g., bone metastasis of cancer). Therefore, an embodiment provides a pharmaceutical composition comprising the liquid composition, for prevention or treatment of osteoporosis, cancer, or cancer metastasis (e.g., bone metastasis of cancer).

The liquid composition or the pharmaceutical composition may be administered to mammals including human beings.

The liquid composition or the pharmaceutical composition may be administered orally or parenterally. For parenteral administration (e.g., injection), an administration route may be selected from intravenous, subcutaneous, intramuscular, intraperitoneal, intradermal, local, intranasal, intrapulmonary, intrarectal, and intratumoral routes, and so forth.

In an embodiment, given the liquid composition comprises an anti-RANKL antibody, the liquid composition or the pharmaceutical composition may be administered intravenously or subcutaneously.

The liquid composition or the pharmaceutical composition may be prepared into formulations suitable for the administration routes.

The liquid composition or the pharmaceutical composition may comprise a pharmaceutically acceptable carrier. The term "pharmaceutically acceptable carrier", as used herein, may refer to at least one selected from solid, semi-solid, or liquid fillers, diluents, encapsulation materials, formulation aids, and any conventional excipient, which are all non-toxic.

### 9. Method for Stabilization of Protein or Method for Preparation of Stabilized Liquid Composition of protein

Another embodiment provides a method of stabilizing a protein or preparing a stabilized liquid composition of a protein, the method comprising a step of mixing the ingredients described above.

An embodiment provides a method of stabilizing a protein or preparing a stabilized aqueous liquid composition of a protein, the method comprising a step of mixing:
(1) the protein with a sugar, a sugar derivative, and/or an amino acid; or
(2) the protein with a sugar, a sugar derivative, and/or an amino acid, and histidine.

When comprising step (1), the method may not comprise mixing at least one selected from an acetate, a succinate, and a glutamate, or mixing a buffer. The method comprising step (1) or step (2), may further comprise mixing a surfactant.

Kinds and amounts of individual ingredients used in the method of stabilizing a protein or preparing a stabilized aqueous liquid composition of a protein are as described above.

### Effect of the Invention

For drug formulations of a protein such as an antibody, optimization is made with respect to kinds and concentrations of buffers, kinds and concentrations of stabilizers, and pH values, thereby achieving protein drug formulations with improved stability of the protein drugs.

### Brief Description of the Drawings

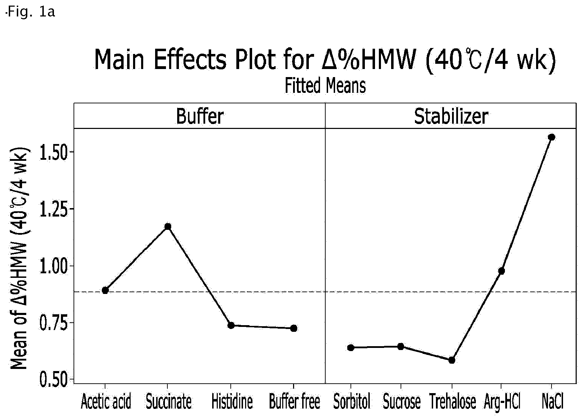

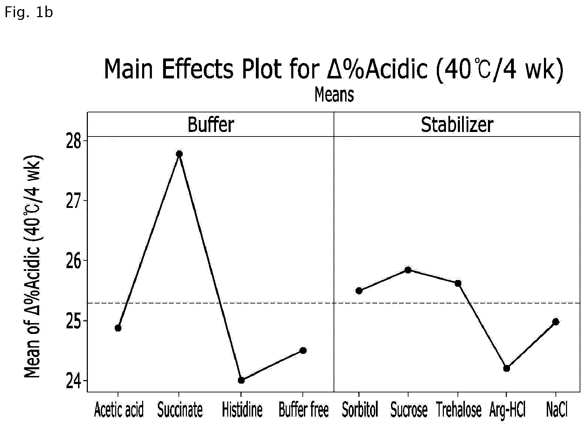

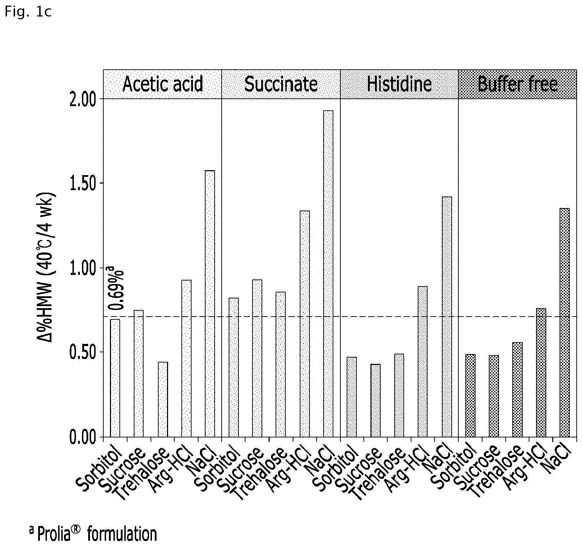

FIG. 1 a is a graph showing average Δ%HMW in protein liquid formulations at 40°C for four weeks as measured in Example 1.2, wherein the left panel shows average Δ%HMW according to kinds of the buffer and the right panel shows average Δ%HMW according to kinds of the stabilizer, and in FIG. 1c, the dotted lines indicates average Δ%HMW (0.69%) of the PROLIA® formulation.
FIG. 1b is a graph showing average Δ%Acidic in protein liquid formulations at 40°C for four weeks as measured in Example 1.2, wherein the left panel shows average Δ%Acidic according to kinds of the buffer and the right panel shows average Δ%Acidic according to kinds of the stabilizer.

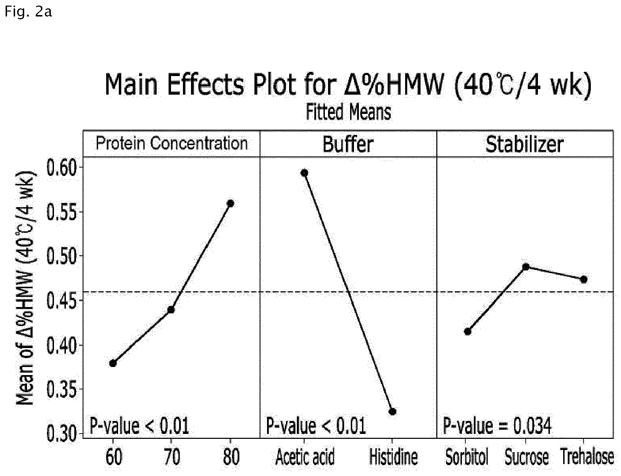

FIG. 2a is a graph showing average Δ%HMW in protein liquid formulations at 40°C for four weeks as measured in Example 2.2, wherein the left panel shows average Δ%HMW according to concentrations of the protein drug, the middle panel shows average Δ%HMW according to kinds of the buffer, and the right panel shows average Δ%HMW according to kinds of the stabilizer.

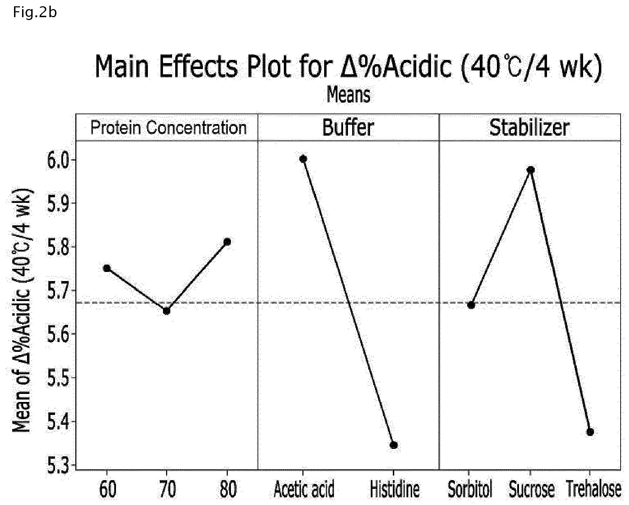

FIG. 2b is a graph showing average Δ%Acidic in protein liquid formulations at 40°C for four weeks as measured in Example 2.2, wherein the left panel shows average Δ%Acidic according to concentrations of the protein drug, the middle panel shows average Δ%Acidic according to kinds of the buffer, and the right panel shows average Δ%Acidic according to kinds of the stabilizer.

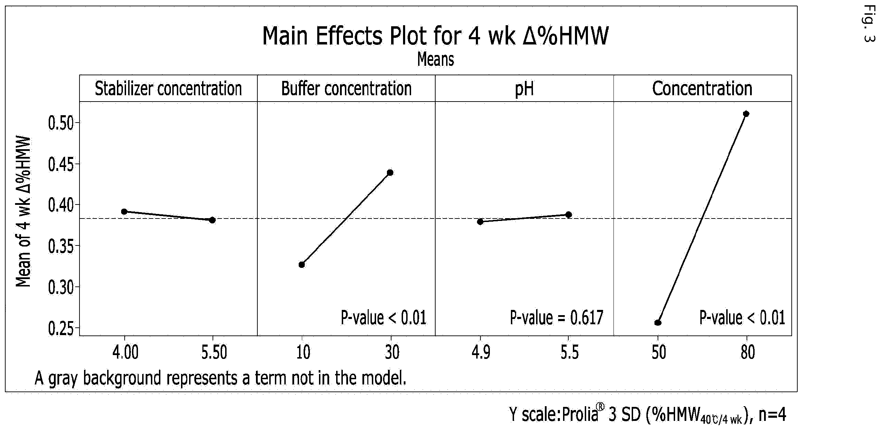

FIG. 3 is a graph showing average Δ%HMW in protein liquid formulations at 40°C for four weeks according to concentrations of the protein drug, kinds of the buffer, kinds of the stabilizer, and pH, as measured in Example 3.

### Mode for Invention

A better understanding of the present disclosure may be obtained through the following examples which are set forth to illustrate, but are not to be construed as limiting the present disclosure.

### EXAMPLE 1: Selection of Buffer/Stabilizer

### 1.1. Preparation of liquid formulation

Liquid formulations with a pH of 5.2, and comprising 60 mg/mL denosumab (Accession No. DB06643), which is an anti-RANKL human monoclonal antibody, as a protein drug, 0.01% polysorbate20 (PS20), buffers and stabilizers as indicated in Table 1 below, were prepared.

**TABLE 1**

| **STD No.** | **Buffer** | **Stabilizer** |
|---|---|---|
| 1-3 | Acetic acid (17mM) | 4.7% Sorbitol |
| 1 | Acetic acid (17mM) | 4.7% Sorbitol |
| 2 | Acetic acid (17mM) | 4.7% Sorbitol |
| 3 | Acetic acid (17mM) | 4.7% Sorbitol |
| 4 | Acetic acid (17mM) | 8% Sucrose |
| 5 | Acetic acid (17mM) | 8% Trehalose |
| 6 | Acetic acid (17mM) | 140 mM Arginine-HCl |
| 7 | Acetic acid (17mM) | 0.8% NaCl |
| 8 | Succinate (17mM) | 4.7% Sorbitol |
| 9 | Succinate (17mM) | 8% Sucrose |
| 10 | Succinate (17mM) | 8% Trehalose |
| 11 | Succinate (17mM) | 140 mM Arginine-HCl |
| 12 | Succinate (17mM) | 0.8% NaCl |
| 13 | Histidine (17mM) | 4.7% Sorbitol |
| 14 | Histidine (17mM) | 8% Sucrose |
| 15 | Histidine (17mM) | 8% Trehalose |
| 16 | Histidine (17mM) | 140 mM Arginine-HCl |
| 17 | Histidine (17mM) | 0.8% NaCl |
| 18 | Buffer free | 4.7% Sorbitol |
| 19 | Buffer free | 8% Sucrose |
| 20 | Buffer free | 8% Trehalose |
| 21 | Buffer free | 140 mM Arginine-HCl |
| 22 | Buffer free | 0.8% NaCl |

| | | |
|---|---|---|
| (1-3: formulations with same composition as PROLIA®; %: w/v) | | |

### 1.2. Measurement of %HMW and %Acidic (40°C condition)

In order to assay stability of the liquid formulations (denosumab 60mg/mL, pH 5.2) comprising various combinations of buffers/stabilizers, prepared in Example 1.1, the liquid formulations were stored at 40°C for four weeks, and high molecular weight (%HMW) and acidic variant amounts (%Acidic) in the liquid formulations were measured by SEC (size-exclusion chromatography) and CEX (cation exchange chromatography), respectively. In detail, %HMW was measured using SEC (size-exclusion chromatography) and %Acidic was measured using CEX (cation exchange chromatography).

In more detail, SEC (size-exclusion chromatography) was conducted using the HPLC system of Waters according to the manufacturer's manual. A total of three peaks are separated according to retention time (molecular weights of proteins). The three peaks correspond to an HMW peak, a monomer peak, and an LMW peak in the order of short retention times (large molecular weights). The substance detected ahead of the monomer is defined as HMW. %HMW was measured using the HPLC (Waters 2695 separation module alliance) and the column (Tosoh, TSK-gel G3000 SWXL) at a flow rate of 0.7 mL/min. under a 24 min injection run time (%HMW = AreaHMw/AreaTOTAL x 100).

In addition, CEX (cation exchange chromatography) was conducted using the HPLC system of Waters according to the manufacturer's manual. A total of three peaks are separated according to retention times (protein charges). The three peaks correspond to an acidic peak, a main peak, and a basic peak in the order of short retention times. A substance detected ahead of the main peak is defined as the acidic peak. %Acidic (acidic variant amount) was measured using the HPLC (Waters 2695 separation module alliance) and the column (Thermo Scientific, Propac WCX-10 column) at a flow rate of 1.0 mL/min under a 32 min injection run time (%Acidic = AreaAcidic/AreaTOTAL x 100).
%HMW (High molecular weight%): %HMW= {area of HMW/area of (HMW + monomer + LMW)}*100;
%Acidic: ratio of acidic variants (%Acidic = {area of acidic/area of (acidic + main + basic)}*100).

The results are summarized in Table 2 for %HMW (%HMW at week 0, %HMW at week 4) and Δ%HMW (%HMW at week 4 - %HMW at week 0) and in Table 3 for %Acidic (%Acidic at week 0, %Acidic at week 4) and Δ%Acidic (%Acidic at week 4 - %Acidic at week 0):

**TABLE 2**

| STD No. | Buffer (No. 1∼17: 17mM) | Stabilizer | %HMW | | |
|---|---|---|---|---|---|
| | | | Week 0 | Week 4 | |
| | | | | %HMW | Δ%HMW |
| 1-3 | Acetic acid | 4.7% Sorbitol | 0.44 | 1.14 | 0.69 |
| | | | N=3, SD:0.04 | N=3, SD:0.07 | N=3, SD:0.09 |
| 1 | Acetic acid | 4.7% Sorbitol | 0.47 | 1.07 | 0.60 |
| 2 | Acetic acid | 4.7% Sorbitol | 0.47 | 1.12 | 0.65 |
| 3 | Acetic acid | 4.7% Sorbitol | 0.39 | 1.22 | 0.83 |
| 4 | Acetic acid | 8% Sucrose | 0.40 | 1.15 | 0.75 |
| 5 | Acetic acid | 8% Trehalose | 0.43 | 0.87 | 0.44 |
| 6 | Acetic acid | 140 mM Arginine-HCl | 0.41 | 1.34 | 0.93 |
| 7 | Acetic acid | 0.8% NaCl | 0.46 | 2.03 | 1.57 |
| 8 | Succinate | 4.7% Sorbitol | 0.42 | 1.24 | 0.82 |
| 9 | Succinate | 8% Sucrose | 0.41 | 1.34 | 0.93 |
| 10 | Succinate | 8% Trehalose | 0.43 | 1.29 | 0.86 |
| 11 | Succinate | 140 mM Arginine-HCl | 0.40 | 1.74 | 1.34 |
| 12 | Succinate | 0.8% NaCl | 0.47 | 2.40 | 1.93 |
| 13 | Histidine | 4.7% Sorbitol | 0.40 | 0.87 | 0.47 |
| 14 | Histidine | 8% Sucrose | 0.40 | 0.83 | 0.43 |
| 15 | Histidine | 8% Trehalose | 0.40 | 0.89 | 0.49 |
| 16 | Histidine | 140 mM Arginine-HCl | 0.28 | 1.17 | 0.89 |
| 17 | Histidine | 0.8% NaCl | 0.42 | 1.84 | 1.42 |
| 18 | Buffer free | 4.7% Sorbitol | 0.53 | 1.02 | 0.49 |
| 19 | Buffer free | 8% Sucrose | 0.51 | 0.99 | 0.48 |
| 20 | Buffer free | 8% Trehalose | 0.50 | 1.06 | 0.56 |
| 21 | Buffer free | 140 mM Arginine-HCl | 0.39 | 1.15 | 0.76 |
| 22 | Buffer free | 0.8% NaCl | 0.50 | 1.85 | 1.35 |

| | | | | | |
|---|---|---|---|---|---|
| (1-3: formulations with the same composition as PROLIA®) | | | | | |

**TABLE 3**

| STD No. | Buffer (No. 1∼17: 17mM) | Stabilizer | %Acidic | | |
|---|---|---|---|---|---|
| | | | Week 0 | Week 4 | |
| | | | | %Acidic | Δ%Acidic |
| 1-3 | Acetic acid | Sorbitol | 17.62 | 25.31 | 7.69 |
| | | | N=3, SD:0.13 | N=3, SD:0.24 | N=3, SD:0.06 |
| 1 | Acetic acid | Sorbitol | 17.78 | 25.42 | 7.64 |
| 2 | Acetic acid | Sorbitol | 17.46 | 25.53 | 8.07 |
| 3 | Acetic acid | Sorbitol | 17.62 | 24.97 | 7.35 |
| 4 | Acetic acid | Sucrose | 18.39 | 25.19 | 6.8 |
| 5 | Acetic acid | Trehalose | 18.01 | 24.9 | 6.89 |
| 6 | Acetic acid | Arginine-HCl | 17.23 | 23.72 | 6.49 |
| 7 | Acetic acid | NaCl | 17.59 | 24.36 | 6.77 |
| 8 | Succinate | Sorbitol | 18.3 | 29.32 | 11.02 |
| 9 | Succinate | Sucrose | 17.91 | 29.26 | 11.35 |
| 10 | Succinate | Trehalose | 17.95 | 28.29 | 10.34 |
| 11 | Succinate | Arginine-HCl | 17.19 | 25.16 | 7.97 |
| 12 | Succinate | NaCl | 18.21 | 26.87 | 8.66 |
| 13 | Histidine | Sorbitol | 18.11 | 23.93 | 5.82 |
| 14 | Histidine | Sucrose | 17.27 | 23.81 | 6.54 |
| 15 | Histidine | Trehalose | 18.14 | 24.01 | 5.87 |
| 16 | Histidine | Arginine-HCl | 17.94 | 23.71 | 5.77 |
| 17 | Histidine | NaCl | 17.76 | 24.67 | 6.91 |
| 18 | Buffer free | Sorbitol | 18.7 | 23.78 | 5.08 |
| 19 | Buffer free | Sucrose | 17.88 | 25.11 | 7.23 |
| 20 | Buffer free | Trehalose | 18.45 | 25.35 | 6.9 |
| 21 | Buffer free | Arginine-HCl | 17.5 | 24.24 | 6.74 |
| 22 | Buffer free | NaCl | 18.4 | 24.08 | 5.68 |

| | | | | | |
|---|---|---|---|---|---|
| (1-3: formulations with the same composition as PROLIA®) | | | | | |

In addition, average Δ%HMW for four weeks is depicted in FIGS. 1a and 1c (Δ%HMW; left: average Δ%HMW according to kinds of the buffer, right: average Δ%HMW according to kinds of the stabilizer, dotted lines of FIG. 1c: average Δ%HMW (0.69%) of PROLIA® formulation) and average Δ%Acidic for four weeks is depicted in FIG. 1b (Δ%Acidic; left: average Δ%Acidic according to kinds of the buffer, right: average %Acidic according to kinds of the stabilizer).

Under the condition of denosumab 60 mg/mL and pH 5.2, as is understood from the data of Tables 2 and 3 and FIGS. 1a and 1b, formulations 13-17, which comprise histidine as a buffer and formulations 18-22, which are free of a buffer, showed lower Δ%HMW and/or lower Δ%Acidic, compared to formulations 1-12, which included an acetic acid or a succinate as a buffer. Lower Δ%HMW and/or lower Δ%Acidic was measured in the formulations containing sorbitol, sucrose, trehalose, or Arg, specifically in the formulations containing sorbitol, sucrose, or trehalose, and more specifically in the formulations containing sorbitol as a stabilizer. These results demonstrate that the formulations that comprise histidine as a buffer or be free of a buffer and comprise sorbitol, sucrose, trehalose, or Arg as a stabilizer allow the protein drug to be more stable, compared to a formulation containing an acetic acid or a succinate as a buffer.

Average values of the high molecular weight (%HMW) and acidic variant amounts (%Acidic) in the formulations comprising various stabilizers (sorbitol, sucrose, trehalose, Arg or NaCl) listed in Tables 2 and 3 are summarized in Table 4, below:

**TABLE 4**

| Average of STD | Buffer (No. 1∼17: 17mM) | Average %HMW | | | Average %Acidic | | |
|---|---|---|---|---|---|---|---|
| | | Week 0 | Week 4 | | Week 0 | Week 4 | |
| | | | %HMW | Δ%HMW | | %Acidic | Δ%Acidic |
| 1-7 | Acetic acid | 0.43 | 1.31 | 0.88 | 17.77 | 24.70 | 6.93 |
| 8-12 | Succinate | 0.43 | 1.60 | 1.18 | 17.91 | 27.78 | 9.87 |
| **13-17** | **Histidine** | **0.38** | **1.12** | **0.74** | **17.84** | **24.03** | **6.18** |
| **18-22** | **Buffer free** | **0.49** | **1.21** | **0.73** | **18.19** | **24.51** | **6.33** |

Under the condition of denosumab 60 mg/mL and pH 5.2, as can be seen in Table 4, formulations 13-17 which comprise histidine as a buffer and formulations 18-22 which are free of a buffer, showed lower average Δ%HMW and lower average Δ%Acidic, compared to formulations 1-12, which comprise an acetic acid or a succinate as a buffer. These results clearly demonstrate that the formulations that comprise histidine as a buffer or be free of a buffer allow the protein drug to be more stable.

### EXAMPLE 2: Stability Depending on Protein Drug Content

### 2.1. Preparation of liquid formulation

Liquid formulations with pH 5.2, comprising the anti-RANKL human monoclonal antibody denosumab at a concentration of 60mg/mL, 70mg/mL, or 80mg/mL, 0.01% polysorbate20 (PS20), a buffer (acetate or histidine), and a stabilizer (sorbitol, sucrose, or trehalose) as listed in Table 5, were prepared.

**TABLE 5**

| STD No. | Protein Conc. | Buffer (17mM) | Stabilizer |
|---|---|---|---|
| 1-3 | 60 mg/mL | Acetic acid | 4.7% Sorbitol |
| 4 | | | 8.0% Sucrose |
| 5 | | | 8.0% Trehalose |
| 6 | | Histidine | 4.7% Sorbitol |
| 7 | | | 8.0% Sucrose |
| 8 | | | 8.0% Trehalose |
| 9-11 | 70 mg/mL | Acetic acid | 4.7% Sorbitol |
| 12 | | | 8.0% Sucrose |
| 13 | | | 8.0% Trehalose |
| 14 | | Histidine | 4.7% Sorbitol |
| 15 | | | 8.0% Sucrose |
| 16 | | | 8.0% Trehalose |
| 17 | 80 mg/mL | Acetic acid | 4.7% Sorbitol |
| 18 | | | 8.0% Sucrose |
| 19 | | | 8.0% Trehalose |
| 20 | | Histidine | 4.7% Sorbitol |
| 21 | | | 8.0% Sucrose |
| 22 | | | 8.0% Trehalose |
| 23-25 | 60 mg/mL | Prolia® | |

| | | | |
|---|---|---|---|
| (1-3: formulations with the same composition as PROLIA®; 9-11: formulations with similar composition to that of Xgeva®; %: w/v) | | | |

### 2.2. Measurement of %HMW and %Acidic (40°C condition)

In order to assay stability of the liquid formulations (pH 5.2) comprising various concentrations of the protein drug (denosumab) and various combinations of buffers/stabilizers, prepared in Example 2.1, the liquid formulations were measured for protein aggregation and acidic variant amounts with reference to Example 1.2 while being stored at 40°C for four weeks.

Measurements are summarized in Table 6 for %HMW (%HMW at week 0, %HMW at week 4) and Δ%HMW (%HMW at week 4 - %HMW at week 0) and in Table 7 for %Acidic (%Acidic at week 0, %Acidic at week 4), and Δ%Acidic (%Acidic at week 4 - %Acidic at week 0):

**TABLE 6**

| STD No. | Protein Conc. | Buffer (17mM) | Stabilizer | %HMW | | |
|---|---|---|---|---|---|---|
| | | | | Week 0 | Week 4 | |
| | | | | | %HMW | Δ%HMW |
| 1-3 | 60 mg/mL | Acetic acid | 4.7% Sorbitol | 0.43 | 0.86 | 0.43 |
| | | | | N=3, SD: 0.03 | N=3, SD: 0.03 | N=3, SD: 0.02 |
| 4 | | | 8.0% Sucrose | 0.43 | 0.93 | 0.50 |
| 5 | | | 8.0% Trehalose | 0.45 | 1.05 | 0.60 |
| 1-5 | | | **Average** | 0.44 | 0.95 | **0.51** |
| 6 | | Histidine | 4.7% Sorbitol | 0.41 | 0.66 | 0.25 |
| 7 | | | 8.0% Sucrose | 0.44 | 0.74 | 0.30 |
| 8 | | | 8.0% Trehalose | 0.46 | 0.74 | 0.28 |
| 6-8 | | | **Average** | 0.44 | 0.71 | **0.28** |
| 9-11 | 70 mg/mL | Acetic acid | 4.7% Sorbitol | 0.47 | 1.04 | 0.57 |
| | | | | N=3, SD: 0.04 | N=3, SD: 0.05 | N=3, SD: 0.08 |
| 12 | | | 8.0% Sucrose | 0.47 | 1.05 | 0.58 |
| 13 | | | 8.0% Trehalose | 0.46 | 0.98 | 0.52 |
| 9-13 | | | **Average** | 0.47 | 1.02 | **0.56** |
| 14 | | Histidine | 4.7% Sorbitol | 0.53 | 0.79 | 0.26 |
| 15 | | | 8.0% Sucrose | 0.48 | 0.81 | 0.33 |
| 16 | | | 8.0% Trehalose | 0.48 | 0.78 | 0.30 |
| 14-16 | | | **Average** | 0.50 | 0.79 | **0.30** |
| 17 | 80 mg/mL | Acetic acid | 4.7% Sorbitol | 0.51 | 1.16 | 0.65 |
| 18 | | | 8.0% Sucrose | 0.49 | 1.24 | 0.75 |
| 19 | | | 8.0% Trehalose | 0.51 | 1.26 | 0.75 |
| 17-19 | | | **Average** | 0.50 | 1.22 | **0.72** |
| 20 | | Histidine | 4.7% Sorbitol | 0.51 | 0.84 | 0.33 |
| 21 | | | 8.0% Sucrose | 0.53 | 1.01 | 0.48 |
| 22 | | | 8.0% Trehalose | 0.48 | 0.88 | 0.40 |
| 20-22 | | | **Average** | 0.51 | 0.91 | **0.40** |
| 23-25 | 60 mg/mL | Prolia® | | 0.61 | 0.98 | 0.36 |
| | | | | N=3, SD: 0.01 | N=3, SD: 0.07 | N=3, SD: 0.08 |

| | | | | | | |
|---|---|---|---|---|---|---|
| (1-3: formulations with the same composition as PROLIA®; 9-11: formulations with similar composition to that of Xgeva®) | | | | | | |

**TABLE 7**

| STD No. | Protein Conc. | Buffer (17mM) | Stabilizer | %Acidic | | |
|---|---|---|---|---|---|---|
| | | | | Week 0 | Week 4 | |
| | | | | | %Acidic | Δ%Acidic |
| 1-3 | 60 mg/mL | Acetic acid | 4.7% Sorbitol | 18.36 | 24.32 | 5.96 |
| | | | | N=3, SD: 0.09 | N=3, SD: 0.10 | N=3, SD: 0.17 |
| 4 | | | 8.0% Sucrose | 18.37 | 24.27 | 5.90 |
| 5 | | | 8.0% Trehalose | 18.21 | 23.70 | 5.49 |
| 1-5 | | | **Average** | 18.31 | 24.10 | **5.78** |
| 6 | | Histidine | 4.7% Sorbitol | 17.96 | 23.31 | 5.35 |
| 7 | | | 8.0% Sucrose | 17.62 | 23.71 | 6.09 |
| 8 | | | 8.0% Trehalose | 18.27 | 23.57 | 5.30 |
| 6-8 | | | **Average** | 17.95 | 23.53 | **5.58** |
| 9-11 | 70 mg/mL | Acetic acid | 4.7% Sorbitol | 18.18 | 24.36 | 6.18 |
| | | | | N=3, SD: 0.12 | N=3, SD: 0.52 | N=3, SD: 0.41 |
| 12 | | | 8.0% Sucrose | 18.01 | 23.68 | 5.67 |
| 13 | | | 8.0% Trehalose | 18.39 | 24.13 | 5.74 |
| 9-13 | | | **Average** | 18.19 | 24.06 | **5.86** |
| 14 | | Histidine | 4.7% Sorbitol | 18.18 | 23.18 | 5.00 |
| 15 | | | 8.0% Sucrose | 18.49 | 23.86 | 5.37 |
| 16 | | | 8.0% Trehalose | 18.37 | 23.28 | 4.91 |
| 14-16 | | | **Average** | 18.35 | 23.44 | **5.09** |
| 17 | 80 mg/mL | Acetic acid | 4.7% Sorbitol | 18.19 | 24.19 | 6.00 |
| 18 | | | 8.0% Sucrose | 17.94 | 24.93 | 6.99 |
| 19 | | | 8.0% Trehalose | 17.98 | 23.76 | 5.78 |
| 17-19 | | | **Average** | 18.04 | 24.29 | **6.26** |
| 20 | | Histidine | 4.7% Sorbitol | 18.04 | 23.25 | 5.21 |
| 21 | | | 8.0% Sucrose | 18.16 | 24.00 | 5.84 |
| 22 | | | 8.0% Trehalose | 18.06 | 23.10 | 5.04 |
| 20-22 | | | **Average** | 18.09 | 23.45 | **5.36** |
| 23-25 | 60 mg/mL | Prolia® | | 20.24 | 27.86 | 7.62 |
| | | | | N=3, SD: 0.20 | N=3, SD: 0.85 | N=3, SD: 0.67 |

| | | | | | | |
|---|---|---|---|---|---|---|
| (1-3: formulations with the same composition as in PROLIA®; 9-11: formulations with similar composition to that of Xgeva®) | | | | | | |

In addition, average Δ%HMW for four weeks is depicted in FIG. 2a (Δ%HMW; left: average Δ%HMW according to concentrations of the protein drug, middle: average Δ%HMW according to kinds of the buffer, right: average Δ%HMW according to kinds of the stabilizer), and average Δ%Acidic for four weeks is depicted in FIG. 2b (Δ%Acidic; left: average Δ%Acidic according to concentrations of the protein drug, middle: average Δ%Acidic according to kinds of the buffer, right: average Δ%Acidic according to kinds of the stabilizer).

As shown in Tables 6 and 7 and FIGS. 2a and 2b, as a whole, the formulations comprising histidine as a buffer guaranteed higher stability of the protein drug, compared to the formulations comprising acetic acid. Inter alia, the formulation comprising the protein drug (denosumab) at a concentration of 60 mg/mL and comprising histidine as a buffer and sorbitol as a stabilizer was found to show the highest stability of the protein drug in particular. In addition, as can be seen in the average value data of Tables 6 and 7, the formulations comprising histidine as a buffer have lower average Δ%HMW and Δ%Acidic values at all the concentrations of the protein drug, specifically, at 60 mg/mL or 70 mg/mL of the protein drug, compared to the formulations comprising acetic acid. More specifically, the average Δ%HMW of the formulations comprising histidine as a buffer was about 0.33% whereas the average Δ%HMW of the formulations comprising acetic acid was 0.59%. In addition, the average Δ%Acidic of the formulations comprising histidine as a buffer was about 5.35%, whereas the average Δ%Acidic of the formulations comprising acetic acid was 5.97%. These results demonstrate that the formulations comprising histidine as a buffer are significantly low in both Δ%HMW and Δ%Acidic and superior in terms of formulation stability at all the protein drug concentrations tested, particularly at 60 mg/mL or 70 mg/mL, compared to the formulations comprising acetic acid.

### EXAMPLE 3: Stability Depending on Stabilizer Concentration, Buffer Concentration, pH and Protein Drug Concentration

Liquid formulations comprising the anti-RANKL human monoclonal antibody denosumab as a protein drug, histidine as a buffer, sorbitol as a stabilizer, and 0.01% polysorbate20 (PS20) as listed in Table 8, below, were prepared.

**TABLE 8**

| **STD No.** | **Protein Conc.** | **pH** | **Histidine Buffer Conc. (mM)** | **Stabilizer Conc. (%)** | **Surfactant Conc. (%)** |
|---|---|---|---|---|---|
| 1 | 50 mg/ml | 4.9 | 10 | 4.0 | |
| 2 | | | | 5.5 | |
| 3 | | | 30 | 4.0 | |
| 4 | | | | 5.5 | |
| 5 | | 5.5 | 10 | 4.0 | |
| 6 | | | | 5.5 | |
| 7 | | | 30 | 4.0 | |
| 8 | | | | 5.5 | |
| 9 | 80 mg/ml | 4.9 | 10 | 4.0 | |
| 10 | | | | 5.5 | 0.01 |
| 11 | | | 30 | 4.0 | |
| 12 | | | | 5.5 | |
| 13 | | 5.5 | 10 | 4.0 | |
| 14 | | | | 5.5 | |
| 15 | | | 30 | 4.0 | |
| 16 | | | | 5.5 | |
| 17 | 65 mg/mL | 5.2 | 20 | 4.75 | |
| 18 | | | | 4.75 | |
| 19 | | | | 4.75 | |

| | | | | | |
|---|---|---|---|---|---|
| (In Table 8, % means %(w/v)) | | | | | |

The liquid formulations thus prepared were measured for Δ%HMW and Δ%Acidic with reference to Example 1.2 while being stored at 40°C for four weeks,

The obtained results are summarized in Table 9 for %HMW (%HMW at week 0, %HMW at week 4) and Δ%HMW (%HMW at week 4 - %HMW at week 0) and in Table 10 for %Acidic (%Acidic at week 0, %Acidic at week 4), and Δ%Acidic (%Acidic at week 4 - %Acidic at week 0):

**TABLE 9**

| STD No. | Protein Conc. | pH | Histidine Buffer Conc. (mM) | Stabilizer Conc. (%) | Surfactant Conc. (%) | %HMW | | |
|---|---|---|---|---|---|---|---|---|
| | | | | | | 0 wk | 4 wk | |
| | | | | | | | %HMW | Δ%HMW |
| 1 | 50 mg/mL | 4.9 | 10 | 4.0 | | 0.27 | 0.48 | 0.21 |
| 2 | | | | 5.5 | | 0.28 | 0.45 | 0.17 |
| 3 | | | 30 | 4.0 | | 0.27 | 0.58 | 0.31 |
| 4 | | | | 5.5 | | 0.26 | 0.62 | 0.36 |
| 5 | | 5.5 | 10 | 4.0 | | 0.31 | 0.51 | 0.20 |
| 6 | | | | 5.5 | | 0.32 | 0.54 | 0.22 |
| 7 | | | 30 | 4.0 | | 0.33 | 0.64 | 0.31 |
| 8 | | | | 5.5 | | 0.33 | 0.62 | 0.29 |
| 9 | 80 mg/mL | 4.9 | 10 | 4.0 | | 0.31 | 0.72 | 0.41 |
| 10 | | | | 5.5 | 0.01 | 0.32 | 0.71 | 0.39 |
| 11 | | | 30 | 4.0 | | 0.31 | 0.93 | 0.62 |
| 12 | | | | 5.5 | | 0.31 | 0.90 | 0.59 |
| 13 | | 5.5 | 10 | 4.0 | | 0.40 | 0.91 | 0.51 |
| 14 | | | | 5.5 | | 0.40 | 0.93 | 0.53 |
| 15 | | | 30 | 4.0 | | 0.39 | 0.95 | 0.56 |
| 16 | | | | 5.5 | | 0.39 | 0.89 | 0.50 |
| 17 | 65 mg/mL | 5.2 | 20 | 4.75 | | 0.33 N=3, SD: 0.00 | 0.70 N=3, SD: 0.01 | 0.37 N=3, SD: 0.01 |
| 18 | | | | 4.75 | | | | |
| 19 | | | | 4.75 | | | | |

**TABLE 10**

| STD No. | Protein Conc. | pH | Histidine Buffer Conc. (mM) | Stabilizer Conc. (%) | Surfactant Conc. (%) | %Acidic | | |
|---|---|---|---|---|---|---|---|---|
| | | | | | | 0 wk | 4 wk | |
| | | | | | | | %Acidic | Δ %Acidic |
| 1 | 50 mg/mL | 4.9 | 10 | 4.0 | 0.01 | 16.98 | 20.73 | 3.75 |
| 2 | | | | 5.5 | | 16.78 | 20.54 | 3.76 |
| 3 | | | 30 | 4.0 | | 17.29 | 21.20 | 3.91 |
| 4 | | | | 5.5 | | 17.31 | 20.72 | 3.41 |
| 5 | | 5.5 | 10 | 4.0 | | 17.56 | 20.63 | 3.07 |
| 6 | | | | 5.5 | | 16.27 | 21.12 | 4.85 |
| 7 | | | 30 | 4.0 | | 17.58 | 21.64 | 4.06 |
| 8 | | | | 5.5 | | 17.08 | 21.70 | 4.62 |
| 9 | 80 mg/mL | 4.9 | 10 | 4.0 | | 16.79 | 20.69 | 3.90 |
| 10 | | | | 5.5 | | 17.20 | 21.11 | 3.91 |
| 11 | | | 30 | 4.0 | | 16.84 | 21.11 | 4.27 |
| 12 | | | | 5.5 | | 17.33 | 20.98 | 3.65 |
| 13 | | 5.5 | 10 | 4.0 | | 17.21 | 21.68 | 4.47 |
| 14 | | | | 5.5 | | 16.61 | 21.42 | 4.81 |
| 15 | | | 30 | 4.0 | | 17.28 | 21.93 | 4.65 |
| 16 | | | | 5.5 | | 17.36 | 21.33 | 3.97 |
| 17 | 65 mg/mL | 5.2 | 20 | 4.75 | | 17.10 N=3, SD: 0.49 | 20.60 N=3, SD: 0.22 | 3.50 N=3, SD: 0.55 |
| 18 | | | | 4.75 | | | | |
| 19 | | | | 4.75 | | | | |

The average Δ%HMW according to protein concentration, buffer concentration, stabilizer concentration, and pH for four weeks, as shown in Tables 9 and 10, are depicted in FIG. 3. As can be seen in FIG. 3, within the tested range, the stability of the protein was increased with decreasing of buffer concentration and protein drug concentration in the formulation. The stabilizer concentrations and pH values were found to have an equivalent level of protein stability in all tested range.

### EXAMPLE 4: Assay for Activity of Denosumab in Liquid Formulation

Liquid formulations with pH 5.2, which comprised 60mg/ml denosumab and 0.01 %(w/v) polysorbate 20 and comprised a buffer and a stabilizer as listed in Table 11 were prepared. In order to assay the activity of the pharmaceutically active ingredient denosumab therein, the formulations were measured for %RBA (Relative Binding Activity) and %RP (Relative Potency Activity) of denosumab against RANKL while being stored at 40°C for four weeks.

%RP (Relative Potency) was measured as follows: Denosumab and RANKL were sequentially loaded into 96-well plates, followed by RANK 293 cells (HEK 293 cell expressing a luciferase and a RANK receptor). After incubation, %RP was analyzed using EnVision® multilabel reader (PerkinElmer, 2015).

%RBA (Relative Binding Activity) was measured as follows: MaxiSorp 96-well plates (Nunc) were coated with RANKL and blocked, followed by loading denosumab thereto. Subsequently, the plates were treated with a secondary antibody and then treated sequentially with a TMB ELISA substance solution and a stop solution. %RBA was analyzed using a microplate reader (SpectraMax 190).

In Table 11, measurements at 40°C of %RBA at week 0 (initial) and week 4 and the variation thereof (Δ%RBA) for 4 weeks, and %RP at week 0 (initial) and week 4 and the variation thereof (Δ%RP) for 4 weeks in the protein liquid formulations are summarized:

**TABLE 11**

| Buffer | Stabilizer | RANKL | | | Neutralization | | |
|---|---|---|---|---|---|---|---|
| | | Initial | 4 wk | | Initial | 4 wk | |
| | | %RBA | %RBA | %ΔRBA | %RP | %RP | %ΔRP |
| 17 mM acetic acid | 4.7%(w/v) Sorbitol | 105 [N=3, SD:1] | 105 [N=3, SD:3] | 0 [N=3, SD:3] | 99 [N=3, SD:0] | 99 [N=3, SD:2] | 0 [N=3, SD:2] |
| | 8.0%(w/v) Trehalose | 106 [N=3, SD:5] | 102 [N=3, SD:2] | -4 [N=3, SD:2] | 97 [N=3, SD:1] | 91 [N=3, SD:4] | -6 [N=3, SD:4] |
| 17 mM histidine | 4.7%(w/v) Sorbitol | 105 [N=3, SD:3] | 107 [N=3, SD:1] | 2 [N=3, SD:1] | 101 [N=3, SD:8] | 106 [N=3, SD:5] | 5 [N=3, SD:5] |
| | 8.0%(w/v) Trehalose | 107 [N=3, SD:5] | 111 [N=3, SD:3] | 4 [N=3, SD:3] | 92 [N=3, SD:4] | 100 [N=3, SD:2] | 8 [N=3, SD:2] |
| Prolia® | | 105 [N=3, SD:5] | 100 [N=3, SD:2] | -5 [N=3, SD:2] | 105 [N=3, SD:2] | 104 [N=3, SD:0] | -1 [N=3, SD:0] |

As shown in Table 11, the formulations comprising histidine as a buffer retained the activity (function) of the protein (denosumab) very well for a relatively long period of time (4 weeks) even under the stress condition of 40°C.

## Claims

1. A liquid composition, which comprises an anti-RANKL antibody, has a pH of 5 to 7 and is free of an acetate.

2. The liquid composition of claim 1, which is free of a succinate.

3. The liquid composition of claim 1 or 2, which is free of glutamate.

4. The liquid composition of any one of claims 1 to 3, comprising histidine.

5. The liquid composition of any one of claims 1 to 4, comprising 1 to 70 mM histidine.

6. The liquid composition of any one of claims 1 to 5, comprising histidine as a protein aggregation inhibitor and/or an acidic variants inhibitor.

7. The liquid composition of any one of claims 1 to 6, wherein as measured following storage at 40°C for four weeks, the liquid composition has variation in high molecular weight (Δ%HMW) of 5.0% or less; or has variation in high molecular weight (Δ%HMW) lower by at least 0.05% than that of the same composition with an exception of comprising at least one selected from the group consisting of an acetate, a succinate, and a glutamate, and/or being free of histidine.

8. The liquid composition of any one of claims 1 to 7, wherein as measured following storage at 40°C for four weeks, the liquid composition has variation in acidic variant amount (Δ%Acidic) of 25.0% or less; or has variation in acidic variant amount (Δ%Acidic) lower by at least 0.05% than that of the same composition with an exception of comprising at least one selected from the group consisting of an acetate, a succinate, and a glutamate and/or being free of histidine.

9. The liquid composition of any one of claims 1 to 8, comprising at least one amino acid selected from the group consisting of alanine, arginine, asparagine, aspartic acid, glutamine, glycine, isoleucine, leucine, lysine, methionine, phenylalanine, proline, serine, threonine, tryptophan, tyrosine, valine, cysteine, and pharmaceutically acceptable salts thereof.

10. The liquid composition of any one of claims 1 to 9, comprising 0.1 mM to 300 mM amino acid.

11. The liquid composition of any one of claims 1 to 10, comprising at least one sugar or sugar alcohol selected from the group consisting of glucose, fructose, galactose, mannose, sucrose, lactose, maltose, trehalose, fructooligosaccharide, galactooligosaccharide, mannanoligosaccharide, starch, glycogen, cellulose, chitin, pectin, aldonic acid, ulosonic acid, uronic acid, aldaric acid, glycerol, erythritol, threitol, arabitol, xylitol, ribitol, mannitol, sorbitol, galactitol, fucitol, iditol, inositol, volemitol, isomalt, maltitol, lactitol, maltotritol, maltotetraitol, and polyglycitol.

12. The liquid composition of any one of claims 1 to 11, comprising 1 to 15 %(w/v) sugar or 1 to 10 %(w/v) sugar alcohol based on the total composition.

13. The liquid composition of any one of claims 1 to 12, comprising a surfactant.

14. The liquid composition of any one of claims 1 to 13, comprising polysorbate 20, polysorbate 80, or a combination thereof, as a surfactant.

15. The liquid composition of any one of claims 1 to 14, comprising 0.001 to 1 %(w/v) surfactant based the total composition.

16. The liquid composition of any one of claims 1 to 15, comprising 50 mg/mL to 80 mg/mL anti-RANKL antibody.

17. The liquid composition of any one of claims 1 to 16, wherein the anti-RANKL antibody is denosumab.

18. The liquid composition of any one of claims 1 to 17, which is applicable to both of an injection agent comprising 60 mg/mL anti-RANKL antibody and an injection agent comprising 70 mg/mL anti-RANKL antibody.

19. A liquid composition, which comprises an anti-RANKL antibody, has a pH of 5 to 7, and is free of a succinate.

20. The liquid composition of claim 19, which is free of an acetate.

21. The liquid composition of claim 19 or 20, which is free of glutamate.

22. The liquid composition of any one of claims 19 to 21, comprising histidine.

23. The liquid composition of any one of claims 19 to 22, comprising 1 to 70 mM histidine.

24. The liquid composition of any one of claims 19 to 23, comprising histidine as a protein aggregation inhibitor and/or an acidic variants inhibitor.

25. The liquid composition of any one of claims 19 to 24, wherein as measured following storage at 40°C for four weeks, the liquid composition has variation in high molecular weight (Δ%HMW) of 5.0% or less; or has variation in high molecular weight (Δ%HMW) lower by at least 0.05% than that of the same composition with an exception of comprising at least one selected from the group consisting of an acetate, a succinate, and a glutamate and/or being free of histidine.

26. The liquid composition of any one of claims 19 to 25 wherein as measured following storage at 40°C for four weeks, the liquid composition has variation in acidic variant amount (Δ%Acidic) of 25.0% or less; or has variation in acidic variant amount (Δ%Acidic) lower by at least 0.05% than that of the same composition with an exception of containing at least one selected from the group consisting of an acetate, a succinate, and a glutamate and/or being free of histidine.

27. The liquid composition of any one of claims 19 to 26, comprising at least one amino acid selected from the group consisting of alanine, arginine, asparagine, aspartic acid, glutamine, glycine, isoleucine, leucine, lysine, methionine, phenylalanine, proline, serine, threonine, tryptophan, tyrosine, valine, cysteine, and pharmaceutically acceptable salts thereof.

28. The liquid composition of any one of claims 19 to 27, comprising 0.1 mM to 300 mM amino acid.

29. The liquid composition of any one of claims 19 to 28, comprising at least one sugar or sugar alcohol selected from the group consisting of glucose, fructose, galactose, mannose, sucrose, lactose, maltose, trehalose, a fructooligosaccharide, a galactooligosaccharide, a mannanoligosaccharide, starch, glycogen, cellulose, chitin, pectin, aldonic acid, ulosonic acid, uronic acid, aldaric acid, glycerol, erythritol, threitol, arabitol, xylitol, ribitol, mannitol, sorbitol, galactitol, fucitol, iditol, inositol, volemitol, isomalt, maltitol, lactitol, maltotritol, maltotetraitol, and polyglycitol.

30. The liquid composition of any one of claims 19 to 29, comprising 1 to 15 %(w/v) sugar or 1 to 10 %(w/v) sugar alcohol based on the total composition.

31. The liquid composition of any one of claims 19 to 30, comprising a surfactant.

32. The liquid composition of any one of claims 19 to 31, comprising polysorbate 20, polysorbate 80, or a combination thereof, as a surfactant.

33. The liquid composition of any one of claims 19 to 32, comprising 0.001 to 1 %(w/v) surfactant based the total composition.

34. The liquid composition of any one of claims 19 to 33, comprising 50 mg/mL to 80 mg/mL anti-RANKL antibody.

35. The liquid composition of any one of claims 19 to 34, wherein the anti-RANKL antibody is denosumab.

36. The liquid composition of any one of claims 19 to 35, which is applicable to both of an injection agent comprising 60 mg/mL anti-RANKL antibody and an injection agent comprising 70 mg/mL anti-RANKL antibody.

37. A liquid composition, which comprises an anti-RANKL antibody, has a pH of 5 to 7, and is free of a buffer.

38. The liquid composition of claim 37, which is free of histidine.

39. The liquid composition of claim 37 or 38, wherein as measured following storage at 40°C for four weeks, the liquid composition has variation in high molecular weight (Δ%HMW) of 5.0% or less; or has variation in high molecular weight (Δ%HMW) lower by at least 0.05% than that of the same composition with an exception of comprising at least one selected from the group consisting of an acetate, a succinate, and a glutamate and/or being free of histidine.

40. The liquid composition of any one of claims 37 to 39, wherein as measured following storage at 40°C for four weeks, the liquid composition has variation in acidic variant amount (Δ%Acidic) of 25.0% or less; or has variation in acidic variant amount (Δ%Acidic) lower by at least 0.05% than that of the same composition with an exception of comprising at least one selected from the group consisting of an acetate, a succinate, and a glutamate and/or being free of histidine.

41. The liquid composition of any one of claims 37 and 40, comprising at least one amino acid selected from the group consisting of alanine, arginine, asparagine, aspartic acid, glutamine, glycine, isoleucine, leucine, lysine, methionine, phenylalanine, proline, serine, threonine, tryptophan, tyrosine, valine, cysteine, and pharmaceutically acceptable salts thereof.

42. The liquid composition of any one of claims 37 to 41, comprising 0.1 mM to 300 mM amino acid.

43. The liquid composition of any one of claims 37 to 42, comprising at least one sugar or sugar alcohol selected from the group consisting of glucose, fructose, galactose, mannose, sucrose, lactose, maltose, trehalose, a fructooligosaccharide, a galactooligosaccharide, a mannanoligosaccharide, starch, glycogen, cellulose, chitin, pectin, aldonic acid, ulosonic acid, uronic acid, aldaric acid, glycerol, erythritol, threitol, arabitol, xylitol, ribitol, mannitol, sorbitol, galactitol, fucitol, iditol, inositol, volemitol, isomalt, maltitol, lactitol, maltotritol, maltotetraitol, and polyglycitol.

44. The liquid composition of any one of claims 37 to 43, comprising 1 to 15 %(w/v) sugar or 1 to 10 %(w/v) sugar alcohol based on the total composition.

45. The liquid composition of any one of claims 37 to 44, comprising a surfactant.

46. The liquid composition of any one of claims 37 to 45, comprising polysorbate 20, polysorbate 80, or a combination thereof, as a surfactant.

47. The liquid composition of any one of claims 37 to 46, comprising 0.001 to 1 %(w/v) surfactant based the total composition.

48. The liquid composition of any one of claims 37 to 47, comprising 50 mg/mL to 80 mg/mL anti-RANKL antibody.

49. The liquid composition of any one of claims 37 to 48, wherein the anti-RANKL antibody is denosumab.

50. The liquid composition of any one of claims 37 to 49, which is applicable to both of an injection agent comprising 60 mg/mL anti-RANKL antibody and an injection agent comprising 70 mg/mL anti-RANKL antibody.
